# EUROPEAN PATENT SPECIFICATION

(11) **EP 1 229 927 B1**
(45) Date of publication and mention of the grant of the patent: **19.12.2007**
(21) Application number: 00972638.1
(22) Date of filing: 27.10.2000
(51) Int. Cl.: A61K 38/27, A61P 3/04

(54) **USE OF A GROWTH HORMONE OR A GROWTH HORMONE SECRETAGOGUE FOR APPETITE-SUPPRESSION OR INDUCTION OF SATIETY**
VERWENDUNG EINES WACHSTUMSHORMONS ODER EINES WACHSTUMSHORMON-SEKRETION FÖRDERNDEN MITTELS ZUR APPETIT-UNTERDRÜCKUNG BZW. SÄTTIGUNGSINDUKTION
UTILISATION D'UNE HORMONE DE CROISSANCE OU D'UN SECRETAGOGUE DE L'HORMONE DE CROISSANCE COMME COUPE-FAIM OU COMME INDUCTEUR DE SATIETE

(30) Priority: 03.11.1999 DK 158599
(43) Date of publication of application: 14.08.2002
(73) Proprietor: NOVO NORDISK A/S, 2880 Bagsvaerd (DK)
(72) Inventor: MALMLÖF, Kjell, S-392 35 Kalmar (SE); JEPSEN, Hanne, DK-3450 Allerød (DK)
(86) International application number: PCT/DK2000/000600
(87) International publication number: WO 2001/032200

(56) References cited:
- EP-A2- 0 363 063
- US-A- 5 952 301
- WILLIAM J. KLISH: 'Childhood obesity: Pathophysiology and treatment' ACTA PAEDIATRICA JAPONICA vol. 37, 1995, pages 1 - 6, XP002937219
- R. VASILATOS-YOUNKEN ET AL.: 'New insights into the mechanism and actions of growth hormone (GH) in poultry' DOMESTIC ANIMAL ENDOCRINOLOGY vol. 17, 1999, pages 181 - 190, XP002937220
- REGINA VASILATOS-YOUNKEN: 'Proposed mechanisms for the regulation of growth hormone action in poultry: Metabolic effects' J. NUTR. vol. 125, 1995, pages 1783S - 1789S, XP002937221
- X. WANG ET AL.: 'Evidence of a role for neuropeptide Y and monoamines in mediating the appetite-suppressive effect of GH' JOURNAL OF ENDOCRINOLOGY vol. 166, 2000, pages 621 - 630, XP002937222

## Description

### FIELD OF INVENTION

The present invention relates to use of growth hormone for the manufacture of a medicament for appetite-suppression or induction of satiety in an adult or mature mammal suffering from obesity.

### BACKGROUND OF THE INVENTION

Growth hormone is a hormone, which stimulates growth of all tissues capable of growing. The growth hormones (GH) from man and from the common domestic animals are proteins of approximately 191 amino acids, synthesized and secreted from the anterior lope of the pituitary gland. Human growth hormone consists of 191 amino acids.

Growth hormone is released from the pituitary. Growth hormone is a key hormone involved in the regulation of not only somatic growth, but also in the regulation of metabolism of proteins, carbohydrates and lipids. The organ systems affected by growth hormone include the skeleton, connective tissue, muscles, and viscera such as liver, intestine, and kidneys. The major effect of growth hormone is to promote growth. Deficiency in growth hormone can result in a number of severe medical disorders, e.g., dwarfism. Furthermore, growth hormone has been proposed for increasing the rate of growth of domestic animals or for decreasing the proportion of fat in animals to be slaughtered for human consumption.

Growth hormone is known to have a number of effects on metabolic processes, e.g., stimulation of protein synthesis and free fatty acid mobilisation and to cause a switch in energy metabolism from carbohydrate to fatty acid metabolism. The main features of the lipolytic effect of growth hormone are rapid increase in circulating levels of free fatty acids (FFA), oxygen consumption and energy expenditure followed by decreases in fat deposits.

Suppression of food intake is important. Obesity is a well-known risk factor for the development of many very common diseases such as atherosclerosis, hypertension, Type II diabetes (non-insulin dependent diabetes mellitus (NIDDM)), dyslipidaemia, coronary heart disease, and osteoarthritis and various malignancies. It also causes considerable problems through reduced motility and decreased quality of life. The incidence of obesity and thereby also these diseases is increasing throughout the entire industrialised world. Except for exercise, diet and food restriction no convincing treatment for reducing body weight effectively and acceptably currently exist. However, due to the important effect of obesity as a risk factor in serious and even mortal and common diseases it will be important to find pharmaceutical compounds useful in prevention and treatment of obesity.

Even mild obesity increases the risk for premature death, diabetes, hypertension, atherosclerosis, gallbladder disease and certain types of cancer. In the industrialised western world the prevalence of obesity has increased significantly in the past few decades. Because of the high prevalence of obesity and its health consequences, its prevention and treatment should be a high public health priority.

When energy intake exceeds energy expenditure, the excess calories are stored in adipose tissue, and if this net positive balance is prolonged, obesity results, i.e. there are two components to weight balance, and an abnormality on either side (intake or expenditure) can lead to obesity.

Studies of the lipolytic effects of GH have been performed during caloric restriction, whereas the direct effects of GH on appetite in obesity are an unexplored area. However, in those human and animal studies where food or energy intake has been measured it has previously been reported that GH has either no effects or that food intake is even slightly increased. From older literature it has been reported that two patients injected with pituitary extracts complained about a slight loss of appetite. Obviously, this was regarded as an adverse event and seen as the result of the uniform experimental diet used. Furthermore, the patients were injected with pituitary extracts (which could contain substances apart from growth hormone) and not with purified growth hormone.

Thus, it has now been found that growth hormone is able to induce satiety, or that growth hormone has a significant appetite reducing effect, in obese adult or nature mammals. It is an object of the present invention to provide for the use of composition for the manufacture of a medicament for the regulation of food intake or for the induction of satiety or for the reduction of appetite in an adult or mature mammal suffering from obesity.

### SUMMARY OF THE INVENTION

It has now been found that growth hormone has an effect on inhibiting food intake (suppressing appetite) in obese adult or mature mammals.

Accordingly, the present invention relates to the use of a growth hormone (GH) for the manufacture of a medicament for appetite suppression in an adult or mature mammal suffering from obesity.

In another aspect, the invention relates to the use of growth hormone for the manufacture of a medicament for the reduction of food intake in an adult or mature mammal suffering from obesity.

In a preferred embodiment, the growth hormone is human growth hormone.

### LIST OF FIGURES

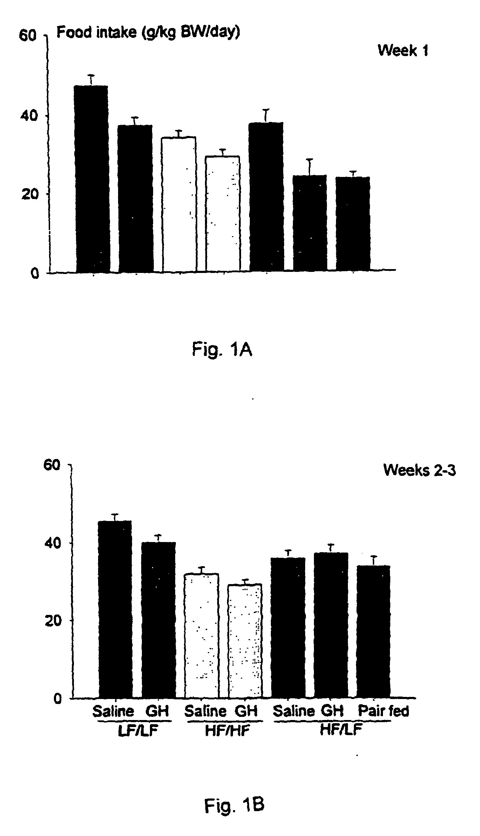

Figure 1A and 1B show the food intake in obese rats following GH treatment

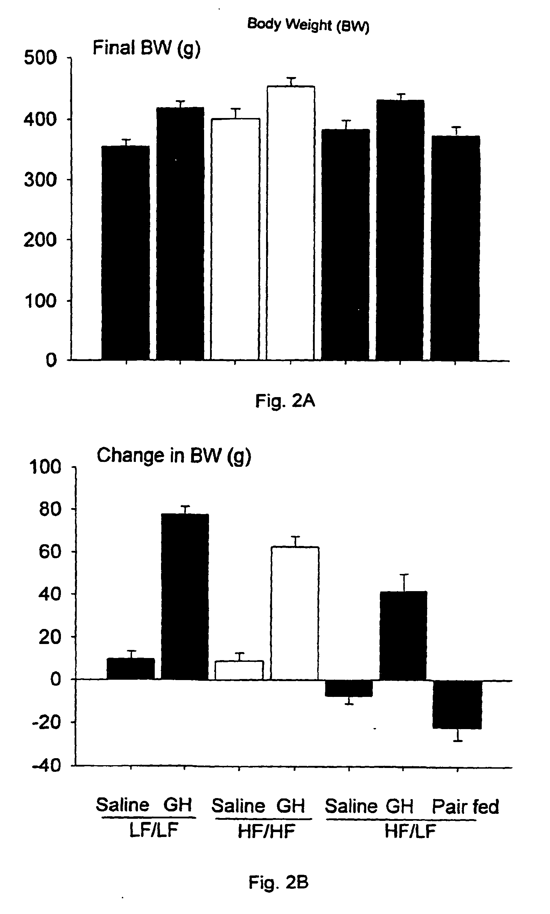

Figure 2A and 2B show the body weight of obese rats following GH treatment

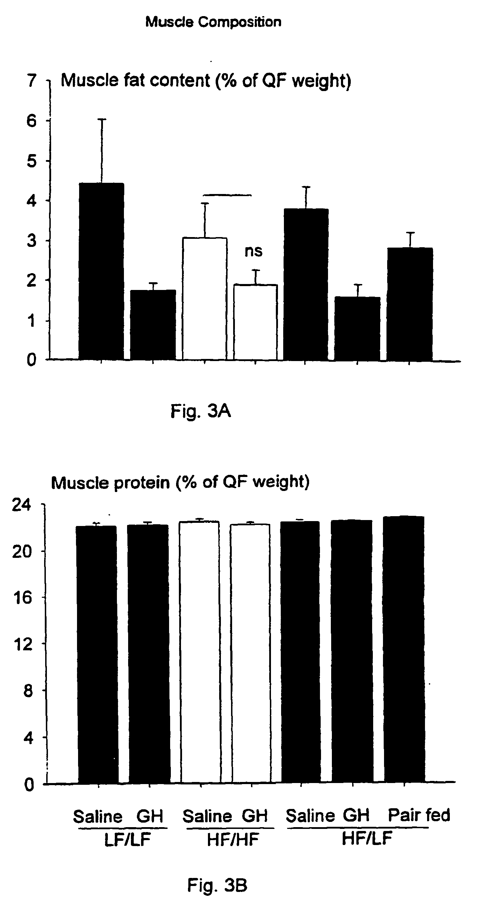

Figure 3A and 3B show the muscle composition of obese rats following GH treatment.

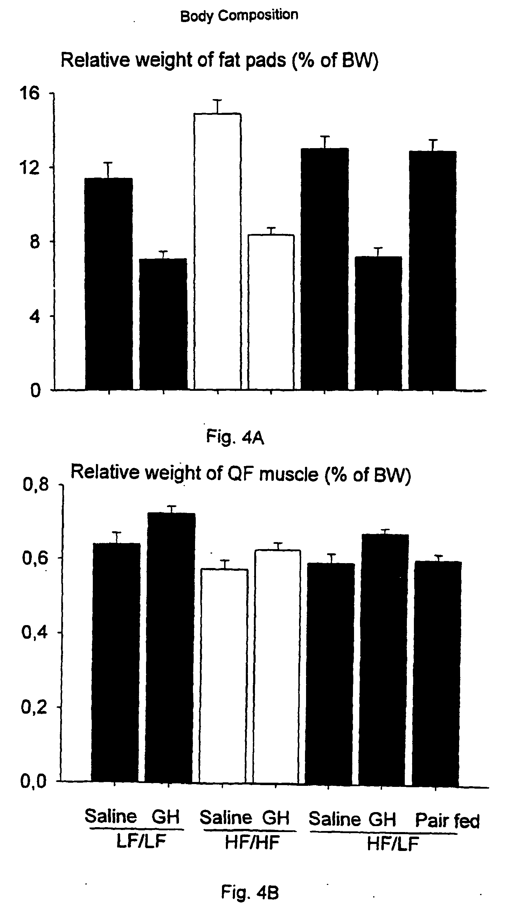

Figure 4A and 4B show the body composition of obese rats following GH treatment.
Figure 5A and 5B show the food intake and body weight of obese rats on HF compared to obese rats on LF.

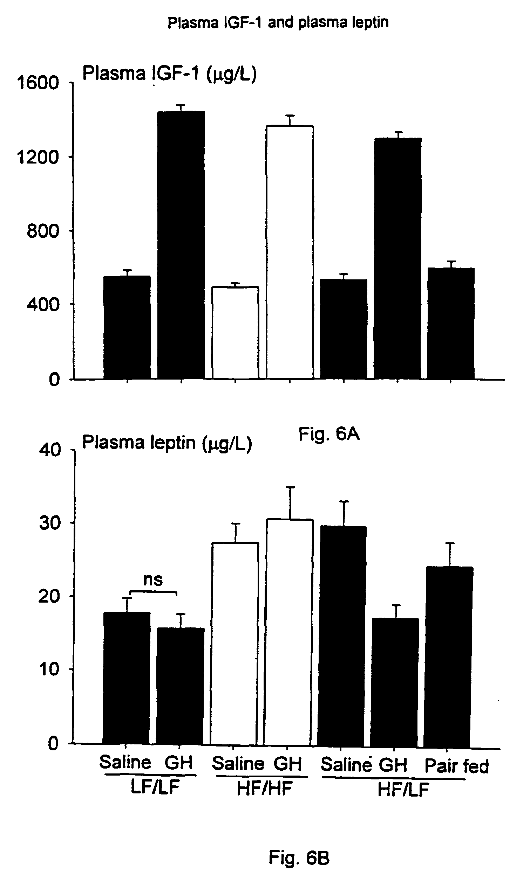

Figure 6A and 6B show the plasma IGF-1 and plasma leptin of obese rats.

### DETAILED DESCRIPTION OF THE INVENTION

### Growth hormone

Growth hormone is a hormone that stimulates growth of all tissues capable of growing. Growth hormone is released from the pituitary. The release is under tight control of a number of hormones and neurotransmitters either directly or indirectly. Growth hormone release can be stimulated by growth hormone releasing hormone (GHRH) and inhibited by somatostatin. In both cases, the hormones are released from the hypothalamus but their action is mediated primarily via specific receptors located in the pituitary. In the present context "growth hormone" may be growth hormone from any origin such as avian, bovine, equine, human, ovine, porcine, salmon, trout or tuna growth hormone, preferably bovine, human or porcine growth hormone, human growth hormone being most preferred. The growth hormone used in accordance with the invention may be native growth hormone isolated from a natural source, e.g. by extracting pituitary glands in a conventional manner, or a growth hormone produced by recombinant techniques, e.g as described in E.B. Jensen and S. Carlsen in Biotech and Bioeng. 36, 1-11 (1990). The "growth hormone derivative" may be a truncated form of growth hormone wherein one or more amino acid residues has (have) been deleted; an analogue thereof wherein one or more amino acid residues in the native molecule has (have) been substituted by another amino acid residue, preferably the residue of a naturally occurring amino acid, as long as the substitution does not have any adverse effect such as antigenicity or reduced action; or a derivative thereof, e.g. deamidated or sulfoxidated forms of the growth hormone or forms having an N- or C-terminal extension such as Met-hGH, Met-Glu-Ala-Glu-hGH or Ala-Glu-hGH. The preferred growth hormone is hGH. Growth hormone mimetics are, for example, peptides, which can dimerise the GH receptor

### Preferred growth hormones

Preferred growth hormones are methionylated human growth hormone (Met-hGH) and human growth hormone (hGH), human growth hormone being most preferred.

In the present context, the term "appetite suppression" is intended to mean any activity or function which causes a decrease in food intake or consumption e.g. by inducing a feeling of satiety or by inhibiting or down-regulating the sensation of hunger.

By the term "low fat diet" or "LF diet" is meant a diet containing less than 15 g/kg fat, preferably less than 10 g/kg fat, more preferred about 5 g/kg fat. By the term "high fat diet" or "HF diet" is meant a diet containing more than 15 g/kg fat, preferably more than 20 g/kg, more preferred more than 25 g/kg. The amount of protein in the LF diet and HF diet may be different or approximately the same. Typically, the content of protein in the two diets is about 15-20 g/kg. Fat content is analyzed as crude fat, all other components are calculated according to official feed tables.

By the term "low fat/high carbohydrate diet" is meant a low fat diet (defined above) containing a relatively low to medium content of protein, e.g., 10-20 g/kg, preferably 15-20 g/kg, and thus containing a relatively high amount of carbohydrate, e.g., more than 50 g/kg. By carbohydrates is meant the sum of polysaccharides and disaccharides.

### Abbreviations

- GH =: growth hormone

### Pharmaceutical administration

The regimen for any patient to be treated with growth hormone as mentioned herein should be determined by those skilled in the art. The daily dose to be administered in therapy can be determined by a physician and will depend on the particular compound employed, on the route of administration and on the age and the condition of the patient. A convenient daily dosage of GH is typically in the range of from about 0.001 mg/kg to about 2.0 mg/kg, preferably from about 0.01 mg/kg to about 1.0 mg/kg. The therapeutic dose of the compound will depend upon the frequency and mode of administration, the sex, age, weight and general condition of the subject treated, the nature and severity of the condition treated and any concomitant diseases to be treated and other factors evident to those skilled in the art.

The GH may be administered in a single dose or it may be administered in repeated doses during the day. Administration should continue until the treated individual is no longer in need of such treatment, for example, until the individual is no longer obese.

The route of administration may be any route that effectively transports the active compound to the appropriate or desired site of action, such as by infusion, injection, topical application or by pulmonal inhalation. Preferred is parenteral (e.g., intramuscular, intraperitonal, intravenous or subcutaneous injection, or implant). The growth hormone can be formulated in dosage forms appropriate for each route of administration. The compositions or dosage forms may appear in conventional forms, for example aerosols, solutions, suspensions or topical applications.

The growth hormone may be administered subcutaneously, intravenously or intramuscularly or it may be administered by continuous or pulsatile infusion. Preferably, the growth hormone is administered subcutaneously.

The composition may be in a form suited for systemic injection or infusion and may, as such, be formulated with a suitable liquid vehicle such as sterile water or an isotonic saline or glucose solution. The compositions may be sterilized by conventional sterilization techniques which are well known in the art. The resulting aqueous solutions may be packaged for use or filtered under aseptic conditions and lyophilized, the lyophilized preparation being combined with the sterile aqueous solution prior to administration. The composition may contain pharmaceutically acceptable auxiliary substances as required to approximate physiological conditions, such as buffering agents, tonicity adjusting agents and the like, for instance sodium acetate, sodium lactate, sodium chloride, potassium chloride, calcium chloride, etc. Examples of liquid carriers are syrup, peanut oil, olive oil, phospholipids, fatty acids, fatty acid amines, polyoxyethylene or water.

It may be of particular advantage to provide the composition of the invention in the form of a sustained release formulation. As such, the composition may be formulated as microcapsules or microparticles containing the growth hormone encapsulated by or dispersed in a suitable pharmaceutically acceptable biodegradable polymer such as polylactic acid, polyglycolic acid or a lactic acid/glycolic acid copolymer.

For nasal administration, the preparation may contain a growth hormone dissolved or suspended in a liquid carrier, in particular an aqueous carrier, for aerosol application. The carrier may contain additives such as solubilizing agents, e.g. propylene glycol, surfactants, absorption enhancers such as lecithin (phosphatidylcholine) or cyclodextrin, or preservatives such as parabenes.

In the pharmaceutical composition of the invention, the growth hormone may be formulated by any of the established methods of formulating pharmaceutical compositions, e.g. as described in Remington: The Science and Practice of Pharmacy (1995).

The growth hormone is advantageous to use in appetite suppression or satiety induction in an adult or mature mammal suffering from obesity.

Within the context of the present invention, "a therapeutic effective growth hormone release" is to be understood as a growth hormone release, which would have a therapeutic effect in treatment of the specific indication, e.g. reduction of food intake in an adult or mature mammal suffering from obesity.

The term obesity implies an excess of adipose tissue. In this context obesity is best viewed as any degree of excess adiposity that imparts a health risk. The distinction between normal and obese individuals can only be approximated, but the health risk imparted by obesity is probably a continuum with increasing adiposity. However, in the context of the present invention, individuals with a body mass index (BMI = body weight in kilograms divided by the square of the height in meters) above 25 are to be regarded as obese.

The term "fat pad" is to be understood as comprising intra-abdominal and peri-renal fat pads or depots.

Within the context of the present invention, treatment is to be understood as treatment and/or prevention.

Furthermore, the compounds according to the invention may be administered in combination with GLP-1, which has been shown to have some effect on appetite suppression (cf. M.D. Turton et al., Nature 379, 4 January 1996, pp. 69-72).

The compounds of the invention may be administered to an adult or mature mammal suffering from obesity, especially a human in need thereof. Such mammals include also animals, both domestic animals, e.g. household Jets, and non-domestic animals such as wildlife.

### EXAMPLES

### Example 1

### Animals:

17 months old Wistar female rats with a live weight of approximately 400 g each were obtained from Møllegård breeding and Research Centre A/S (Denmark). They were kept in conventional plastic cages. In each cage 2 animals were kept together throughout the experiment.

### Feed

The low fat diet (LF-diet, Table 1) and high fat diet (HF-diet, Table 1) were given to groups of rats for about 3 months (diet before test period, table 2). Thereafter, rats were randomly assigned to smaller groups of 10-12 animals to receive diets and treatments according to table 2, and the test period with active dosing was stared. During the test period the consumption of food was registered every day during three weeks.

**Table 1.**

| Composition of experimental diets | | | | | |
|---|---|---|---|---|---|
| | | **Content** | | | |
| **Diet** | **Abbreviation** | **Protein g/kg** | **Fat g/kg** | **Carbohydrates g/kg** | **Energy Mcal/kg** |
| Low Fat | LF | 160 | 55^{a} | 540^{b} | 3.2 |
| High Fat | HF | 160 | 320^{a} | 330^{b} | 4.8 |

| | | | | | |
|---|---|---|---|---|---|
| a: fat content was analysed as crude fat, all other components were calculated according to official food tables. b: the sum of polysaccharides and disaccharides | | | | | |

### Test period

The animals were treated for three weeks between 24^{th} November and 15^{th} December 1998.

### Weight

The animals were weighed at the start of the test period (day 0), at days 2, 7 and 14 and at the end of the experiment (day 21).

### Test compounds

Human growth homone (hGH); rat growth hormone (rGH); growth hormone secretagogue 5-amino-5-methyl-hex-2-enoic acid N-methyl-N-((1R)-1-(methyl-((1R)-1-(methylcarbamoyl)-2-phenylethyl)carbamoyl)-2-(naphthalen-2-yl)ethyl)amide (compound I)

### Dosing

Compound (I) : 30 mg/kg/day (administrated in the morning, at about 07.00 h) hGH: 4 mg/kg/day (2 mg/kg x 2) injected in the morning at about 07.00 h and in the evening, at about 14.00 h.

rGH: 4 mg/kg/day (2 mg/kg x 2) injected in the morning at about 07.00 h and in the evening, at about 14.00 h.

Solute for compound (I) was: 0.9% saline. Growth hormone, both human and rat growth hormone, was first dissolved in small amount of distilled water and then further diluted in 0.9 % saline. The proportions were approximately 1:5.

### Injection volume

rGH or hGH were administered subcutaneously (sc.) (0.5 ml/injection). Animals in groups with no active growth hormone treatment were injected with saline. Compound (I) was administered directly into the stomach (po.) via a stomach tube. The dose volume was 0.5 ml/animal. Animals not receiving active compound (I) treatment were treated with saline.

### Effect variable and statistical analyses

Food consumption is expressed both as grams of diet consumed per day (g/d) and grams of diet consumed per kg body weight per day (g/kg/d).

The statistical analysis was performed using analysis of variance, in which group was regarded the main source of variation. Calculations were executed in the GLM (General linear model) procedure of the SAS (Statistical analyses system) program.

### Results (Table 2)

When the consumption of food was compared between the four groups (groups 3, 4, 5, 6) which were switched over from the HF-diet to the LF-diet big differences were found. These differences were particularly prominent directly at start of the test period. Thus, during the first two days, animals in group 3 which had only been made to switch diet consumed 28.2 g/kg/d. In animals which in parallel were given either of hGH or rGH, the food consumption fell dramatically and unexpectedly down to 15.9, 10.0 g/kg/day, respectively (groups 4,5). The effect of compound (I) (Group 6) seemed to mimic the effect of hGH and rGH.

At termination of the experiment (day 21) circulating plasma levels of insulin-like growth factor-1 (IGF-1) were analysed. Animals in groups treated with hGH and rGH (groups 4 and 5) were found to have mean levels of 772 and 790 ng/ml, respectively, whereas the non-GH-treated animals in group 3 had a significantly lower level of 333 ng/ml. This value was also significantly lower than what was in animals treated with compound (I) that had a mean level of 477 ng/ml.

**Table 2**

| Daily food consumption during the first 2 days of treatment. Data represent means. The means are based on the mean consumption of 2 animals contained in each cage. | | | | | | |
|---|---|---|---|---|---|---|
| **Group** | **n** | **Diet before test period** | **Diet during test period** | **Treatment** | **Food Consumption (g/d)** | **Food consumption (g/kg/d)** |
| Group 1 | 6 | HF | HF | saline sc. | 14.0 | 32.7 |
| | | | | saline po. | | |
| Group 2 | 5 | LF | LF | saline sc. | 17.6 | 45.6 |
| | | | | saline po. | | |
| Group 3 | 6 | HF | LF | saline sc. | 12.3 | 28.2 |
| | | | | saline po. | | |
| Group 4 | 6 | HF | LF | hGH sc. | 6.9 | 15.9 |
| | | | | saline po. | | |
| Group 5 | 6 | HF | LF | rGH sc. | 4.0 | 10.0 |
| | | | | saline po. | | |
| Group 6 | 5 | HF | LF | saline sc. | 10.2 | 23.6 |
| | | | | **Compound (I)** po. | | |

In obese rats growth hormone induces satiety and decreases appetite.

### Example 2

In an other experiment the conditions were very similar to those in example 2, but in this experiment hGH was dosed also to animals who continued on the HF-diet and the LF diet, groups 2 and 4 respectively. The idea was to investigate whether shift of diet was a necessary condition for GH to evoke satiety and decrease of appetite.

### Table 3

Daily food consumption during the first 7 days of treatment. Data represent means. The means are based on the mean consumption of 2 animals contained in each cage.

| **Group** | **n** | **Diet before test period** | **Diet during test period** | **Treatment** | **Food consumption (g/d)** | **Food consumption (g/kg/d)** |
|---|---|---|---|---|---|---|
| Group 1 | 6 | HF | HF | saline sc. | 13.3 | 33.9 |
| | | | | saline po. | | |
| Group 2 | 6 | HF | HF | hGH sc. | 11.7 | 28.0 |
| | | | | saline po. | | |
| Group 3 | 6 | LF | LF | saline sc. | 16.5 | 47.2 |
| | | | | saline po. | | |
| Group 4 | 6 | LF | LF | hGH sc. | 13.3 | 35.5 |
| | | | | saline po. | | |
| Group 5 | 6 | HF | LF | saline sc. | 14.3 | 37.7 |
| | | | | saline po. | | |
| Group 6 | 6 | HF | LF | hGH sc. | 9.1 | 23.7 |
| | | | | saline po. | | |

The shift of diet as performed in example 2 does not seem to be a necessary prerequisite for GH to express its anorectic effect. Instead, GH induces satiety and decreases appetite during a number of dietary conditions.

### Example 3

Composition of high fat (HF) and low fat (LF) diets:

| Ingredients, g/kg | HF | LF |
|---|---|---|
| Com meal | 493 | 818 |
| Wheat bran | 27 | 27 |
| Casein | 148 | 110 |
| Animal fat | 300 | 13 |
| Vitamins and minerals | 32 | 32 |
| | | |

| Chemical composition, g/kg | | |
|---|---|---|
| Crude protein | 170 | 170 |
| Crude fat | 320 | 50 |
| Metabolizable energy, Mcal/kg | 4.8 | 3.2 |

GH reduces food intake and fat pad weight in obese and aged female rats. This study was performed to elucidate if these responses could be could be modulated by type of diet.

### Experimental procedures

High fat (HF) or low fat (LF) diets were given to 12 months old female rats for 14 weeks. Thereafter, dosing began and a number of animals were shifted from the HF to the LF diet, others continued as before according to the scheme below. Saline or human GH (hGH), in a total dose of 4 mg/kg/d, was injected subcutaneously, twice a day. After three weeks of treatment animals were sacrificed by decapitation, blood was collected and tissues were quickly excised.

The fattening period was 14 weeks; the dosing period was 3 weeks.

The number of rats on HF during the fattening period was 58. The number of rats on LF during the fattening period was 23.

Of the 58 HF rats, 23 remained on HF diet during dosing period, and 35 shifted to LF diet at start of and during dosing period. Of the 23 LF rats, 23 remained on LF diet during dosing period.

**Table 4**

| **Group** | **Diet during fattening period (14 weeks)** | **Diet during dosing period (3 weeks)** | **Compound administered** |
|---|---|---|---|
| 1 | HF: 58 rats | 12 rats continuing on HF (HF/HF) | saline |
| 2 | | 11 rats continuing on HF (HF/HF) | hGH |
| 3 | | 11 rats shifting to LF (HF/LF) | Saline |
| 4 | | 12 rats shifting to LF (HF/LF) (pair fed with 5) | hGH |
| 5 | | 12 rats shifting to LF (HF/LF) (pair fed with 4) | Saline |
| | | | |
| 6 | LF: 23 rats | 12 rats continuing on LF (LF/LF) | hGH |
| 7 | | 11 rats continuing on LF (LF/LF) LF/LF | Saline |

**Table 5**

| | **Food Intake (g/kg BW/day)** | | **Body Weight (BW)** | |
|---|---|---|---|---|
| **Group** | **Week 1 (Fig. 1A)** | **Week 2-3 (Fig. 1B)** | **Final BW (g) (Fig.2A)** | **Change in BW (g) (Fig. 2B)** |
| Group 7 | 49 | 45 | 350 | 10 |
| Group 6 | 38 | 40 | 420 | 78 |
| Group 1 | 33 | 31 | 400 | 10 |
| Group 2 | 28 | 28 | 450 | 62 |
| Group 3 | 37 | 35 | 380 | -5 |
| Group 4 | 23 | 36 | 430 | 42 |
| Group 5 | 22 | 32 | 370 | -22 |

**Table 6**

| | **Muscle Composition** | | **Body Composition** | |
|---|---|---|---|---|
| **Group** | **Muscle fat content (% of QF weight) (Fig. 3A)** | **Muscle protein (% of QF weight) (Fig. 3B)** | **Relative weight of fat pads (% of BW) (Fig. 4A)** | **Relative weight of QF muscle (% of BW) (Fig. 4B)** |
| Group 7 | 4.4 | 22.0 | 11.5 | 0.65 |
| Group 6 | 1.7 | 22.0 | 7.0 | 0.75 |
| Group 1 | 3.1 | 23.0 | 15.0 | 0.55 |
| Group 2 | 1.9 | 22.0 | 8.5 | 0.65 |
| Group 3 | 3.8 | 22.5 | 13.0 | 0.60 |
| Group 4 | 1.6 | 22.5 | 7.0 | 0.70 |
| Group 5 | 2.9 | 23.0 | 13.0 | 0.60 |

| | | | | |
|---|---|---|---|---|
| QF = Quadriceps femoris | | | | |

**Table 7**

| | | | | |
|---|---|---|---|---|
| **Group** | **Plasma IGF-1 (µg/L) (Fig. 6A)** | **Plasma leptin (µg/L) (Fig. 6B)** | | |
| Group 7 | 480 | 17 | | |
| Group 6 | 1500 | 15 | | |
| Group 1 | 450 | 27 | | |
| Group 2 | 1400 | 31 | | |
| Group 3 | 550 | 29 | | |
| Group 4 | 1300 | 17 | | |
| Group 5 | 600 | 25 | | |

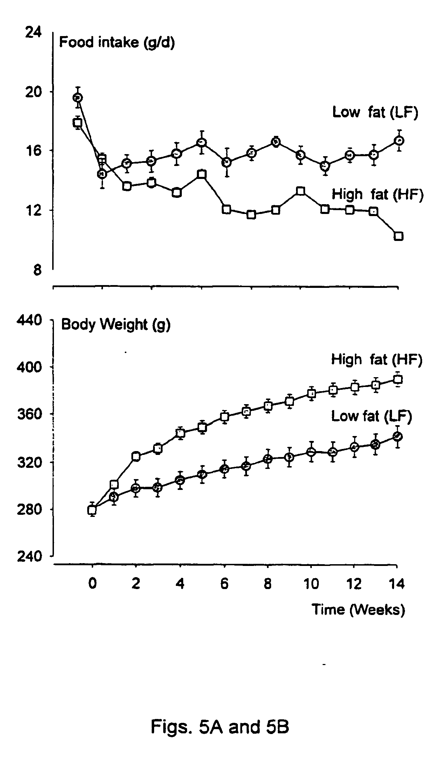

The food intake during the 14 weeks of fattening prior to administration of GH or saline can be seen in Fig. 5A and 5B.

Fig. 5A shows the food intake (g/day) of the rats on HF diet and of the rats on LF diet.

Fig. 5B shows the body weight (g) of the rats on HF diet and of the rats on LF diet.

GH dramatically decreases fat pad weight irrespective of diet and the degree of obesity of aged female rats. GH also reduces food intake. Pair fed animals, although losing body weight, do not show the same decrease in fat pad weight as their GH treated mates. It can thus be concluded that GH exerts specific effects on adipose tissue that either precedes or are separated from its effect on food intake.

## Claims

1. Use of a growth hormone (GH) for the manufacture of a medicament for reducing food intake in an adult or mature mammal suffering from obesity.

2. The use according to claim 1, wherein said medicament suppresses appetite or induces satiety in said mammal.

3. The use according to claim 1 or 2, wherein said mammal is a human, and said growth hormone is human growth hormone (hGH).

## Patentansprüche

1. Verwendung eines Wachstumshormons (growth hormone; GH) zur Herstellung eines Medikaments zum Reduzieren der Nahrungsaufnahme bei einem erwachsenen oder vollentwickelten Säuger, der an Fettsucht leidet.

2. Verwendung nach Anspruch 1, wobei das Medikament den Appetit des Säugers unterdrückt oder eine Sättigung bei dem Säuger herbeiführt.

3. Verwendung nach Anspruch 1 oder 2, wobei der Säuger ein Mensch ist und das Wachstumshormon Humanwachstumshormon (hGH) ist.

## Revendications

1. Utilisation d'une hormone de croissance (GH) pour la fabrication d'un médicament pour réduire l'absorption de nourriture chez un mammifère adulte ou à maturité souffrant d'obésité.

2. L'utilisation selon la revendication 1, où ledit médicament coupe l'appétit ou provoque la satiété chez ledit mammifère.

3. L'utilisation selon l'une des revendications 1 ou 2, où ledit mammifère est un humain et ladite hormone de croissance est l'hormone de croissance humaine (hGH).
